(19) Europäisches Patentamt
European Patent Office
Office européen des brevets

(11) EP 0 913 483 A2

(12) **EUROPÄISCHE PATENTANMELDUNG**

(43) Veröffentlichungstag:
**06.05.1999 Patentblatt 1999/18**

(21) Anmeldenummer: **98120725.1**

(22) Anmeldetag: **31.10.1998**

(51) Int. Cl.$^6$: **C12Q 1/50**, C12Q 1/48, C12Q 1/32, C12Q 1/533, C12Q 1/44

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **03.11.1997 DE 19748490**

(71) Anmelder: **Roche Diagnostics GmbH**
**68305 Mannheim (DE)**

(72) Erfinder:
- **Siedel, Joachim**
**82327 Tutzing (DE)**
- **Nagel, Rolf**
**68642 Bürstadt (DE)**

(54) **Verfahren und Reagenz zur Creatin-Kinase-Bestimmung**

(57) Verfahren bzw. Reagenz zur photometrischen, enzymatischen Bestimmung von Creatin-Kinase (CK) in biologischem Probenmaterial durch Bildung von ATP aus Creatinphosphat und ADP und Nachweis des gebildeten ATP in Gegenwart einer von Glucose unterschiedlichen Hexose, einer geeigneten Hexokinase und Hexose-6-Phosphat-Dehydrogenase und gegebenenfalls mittels weiterer Folgereaktionen. Ein entsprechendes Reagenz in flüssiger Form ist bei 2 bis 8°C bis zu 12 Monaten ohne signifikanten Funktionsverlust stabil.

EP 0 913 483 A2

## Beschreibung

Die Erfindung betrifft ein verbessertes Verfahren bzw. stabilisiertes Reagenz zur photometrischen Bestimmung der Creatin-Kinase in biologischen Proben, wie insbesondere menschlichem Blut-Serum oder -Plasma. Das Verfahren bzw. das Reagenz ist im wesentlichen dadurch gekennzeichnet, daß eine von Glucose unterschiedliche, enzymatisch phosphorylierbare Hexose zugegen ist.

Die Bestimmung der Creatin-Kinase in Serum oder Plasma spielt eine wichtige Rolle bei der Diagnose des Herzinfarkts. Als Standard-Methode wird dazu ein photometrischer Test verwendet, bei dem in gekoppelten Enzymreaktionen aus Creatinphosphat und Adenosin-5'-diphosphat (ADP) durch die in der Probe enthaltene Creatin-Kinase (CK) neben Creatin Adenosin-5'-triphosphat (ATP) erzeugt wird; mit ATP wird aus Glucose in Gegenwart von Hexokinase (HK) Glucose-6-phosphat gebildet, das in der von der Glucose-6-phosphat-Dehydrogenase (G6P-DH) katalysierten Reaktion unter gleichzeitiger Umwandlung von $NAD^+$ oder $NADP^+$ in NADH bzw. NADPH zu Gluconat-6-phosphat oxidiert wird:

$$\text{Creatinphosphat} + \text{ADP} \xrightarrow[\text{Mg}^{++}]{\text{Creatin - Kinase}} \text{Creatin} + \text{ATP}$$

$$\text{ATP} + \text{Glucose} \xrightarrow[\text{Mg}^{++}]{\text{Hexokinase}} \text{Glucose-6-phosphat} + \text{ADP}$$

$$\text{Glucose-6-phosphat} + \text{NAD(P)}^+ \xrightarrow{\text{G6P - DH}} \text{Gluconat-6-phosphat} + \text{NAD(P)H} + \text{H}^+$$

Als Meßgröße dient die in einem vorgegebenen Zeitintervall bei einer bestimmten Temperatur, üblicherweise zwischen 25 und 37 °C, durch die Bildung von NAD(P)H erfolgende Extinktionszunahme, die der CK-Aktivität im Probevolumen proportional ist. Damit die CK ihre volle enzymatische Aktivität entfalten kann, wird die Bestimmung vorzugsweise in Gegenwart von CK-Aktivatoren, beispielsweise von Thiol-Verbindungen wie insbesondere N-Acetylcystein, durchgeführt.

Weiterhin werden dem dem CK-Test zugrundeliegenden Reagenz bevorzugt zusätzlich Inhibitoren für eventuell in der Probe vorhandene Adenylat-Kinase, wie beispielsweise Adenosin-5'-monophosphat (AMP) und/oder Di-adenosin-pentaphosphat zugesetzt. Andernfalls kann entsprechend folgender Reaktion:

$$\text{ADP} + \text{ADP} \xrightarrow[\text{Mg}^{++}]{\text{Adenylat - Kinase}} \text{ATP} + \text{AMP}$$

aus ADP unspezifisch ATP entstehen. Dies kann zu einer zusätzlichen, die CK-Bestimmung verfälschenden NAD(P)H-Bildung führen.

Für die Bestimmung von Creatin-Kinase-Isoenzymen können dem Reagenz des weiteren noch spezifische Inhibitoren, wie beispielsweise gegen bestimmte CK-Isoenzyme gerichtete Antikörper zugesetzt werden.

Zur Steigerung der Nachweisempfindlichkeit kann das Reagenz wahlweise auch noch 6-Phosphogluconolactonase und Gluconat-6-phosphat-Dehydrogenase enthalten; in diesem Fall werden pro Mol gebildetem ATP bzw. Glucose-6-phosphat zwei Mol NADH bzw. NADPH erzeugt (R. Vormbrock und R. Helger, Enzyme <u>38</u>, Suppl. 1 (1987), S. 20/21).

Grundsätzlich können sämtliche für die CK-Bestimmung erforderlichen Komponenten, d.h. Enzyme und Substrate, in einem einzigen Reagenz vorliegen. Insbesondere an Analysengeräten wird die Bestimmung jedoch bevorzugt dergestalt durchgeführt, daß man die Probe zunächst mit einem ersten, außer Creatin-Phospat sämtliche für die Nachweisreaktion erforderlichen Komponenten und mögliche weitere Hilfsstoffe, wie beispielsweise N-Acetylcystein, Adenylat-Kinase-Inhibitoren sowie gegebenenfalls CK-Isoenzym-Inhibitoren, enthaltenden Teilreagenz für einige Minuten vorinkubiert und anschließend die Nachweisreaktion durch Zugabe eines zweiten, im wesentlichen Creatinphosphat in gepufferter Lösung enthaltenden Teilreagenzes startet.

Neben der Vermeidung einer durch die CK hervorgerufenen NAD(P)H-Bildung in der Phase der in Gegenwart der zugesetzten Thiol-Verbindung ablaufenden Aktivierung des Enzyms ist es auf diese Weise außerdem möglich, etwaige Verfälschungen des Meßergebnisses durch überhöhte, nicht mehr ausreichend durch Adenvlat-Kinase-Inhibitoren hemmbare Aktivitäten an Adenylat-Kinase, wie sie beispielsweise in hämolytischen Proben auftreten können, zu verhindern.

Hierzu wird nach Vermischen von Probe und dem ersten Teilreagenz eine erste Messung der Bildungsgeschwindigkeit an NAD(P)H durchgeführt und das Ergebnis von der NAD(P)H-Bildungsgeschwindigkeit nach Zugabe des zweiten, Creatinphosphat-haltigen Teilreagenzes abgezogen.

Ein Nachteil für den Anwender solcher Reagenzien besteht nun insbesondere darin, daß die Reagenzien vor Gebrauch erst durch Auflösen von festen Bestandteilen, wie z.B. Lyophilisaten, Granulaten oder Tabletten, hergestellt werden müssen und darüber hinaus selbst bei Kühllagerung zwischen etwa 2 bis 8 °C nur wenige Tage oder Wochen haltbar sind. In EP 0 774 514 sind zwar Stabilisierungsmaßnahmen für entsprechende Flüssigreagenzien zur Bestimmung von CK beschrieben, wie beispielsweise der Zusatz eines Phosphins und einer Sulfhydrylverbindung, die angegebenen Stabilitäten genügen den heutigen Anforderungen jedoch nicht in vollem Umfang.

Demzufolge und insbesondere um die Arbeiten im klinischen Labor weiter zu rationalisieren, besteht heute mehr und mehr ein Bedarf an Reagenzien, die in gebrauchsfertiger Form über mindestens 12 Monate bei 2 bis 8 °C lagerfähig und stabil sind.

Beim Versuch, ein entsprechendes flüssig-stabiles Reagenz für die Creatin-Kinase-Bestimmung herzustellen, wurde festgestellt, daß die Glucose-6-phosphat-Dehydrogenase auch in Abwesenheit von ATP die eigentlich als Substrat für die Hexokinase-Reaktion dienende Glucose langsam, aber kontinuierlich unter NAD(P)H-Bildung umsetzt. Darüber hinaus gelang es auch nicht, diese Glucose-Dehydrogenase-Nebenaktivität des Enzyms durch weitere Enzymreinigungsschritte entscheidend zu verringern oder gar völlig auszuschalten.

Abgesehen von dem unerwünschten vorzeitigen Verbrauch an nicht reduziertem Coenzym zeigte es sich insbesondere, daß sich das gebildete NAD(P)H bzw. daraus während der Reagenzlagerung entstehende Abbau- oder Folgeprodukte hemmend auf die Nachweisreaktion auswirken und dadurch zu falsch niedrigen Creatin-Kinase-Werten führen können.

Es besteht daher der Bedarf an einem Verfahren bzw. Reagenz zur enzymatischen Bestimmung der Creatin-Kinase, das im wesentlichen die eingangs erwähnten Enzymreaktionen umfaßt, ohne daß es jedoch zu einer vorzeitige Bildung von NAD(P)H kommt.

Diese Aufgabe wird erfindungsgemäß dadurch gelöst, daß man entweder dem gesamten CK-Reagenz oder mindestens einem von mehreren Teilreagenzien, wie beispielsweise dem für die Vorinkubation bzw. zur Bestimmung der Adenylat-Kinase verwendeten Teilreagenz anstelle von Glucose eine von Glucose unterschiedliche, von einer Hexokinase phosphorylierbare Hexose zusetzt. Das durch die entsprechende Hexose entstehende Hexose-6-phosphat kann entweder direkt mit einer entsprechenden Hexose-6-Phosphat-Dehydrogenase bestimmt werden; besonders bevorzugt wird es jedoch nach enzymatischer Isomerisierung als Glucose-6-phosphat bestimmt. Erfindungsgemäß kommt als Hexose-Verbindung jede von Glucose unterschiedliche Aldo- oder Ketohexose, wie beispielsweise Allose, Altrose, Mannose, Gulose, Idose, Galactose, Talose, Psicose, Fructose, Sorbose oder Tagatose in Betracht. Erfindungsgemäß wird als Hexose bevorzugt Fructose oder/und Mannose verwendet. Im Falle der Verwendung von Fructose wird als isomerisierendes Enzym Phosphoglucose-Isomerase (PGI, EC 5.3.1.9), im Falle der Verwendung von Mannose eine Kombination aus Phosphomannose-Isomerase (PMI, EC 5.3.1.8) und PGI eingesetzt. Der diesem Verfahren zugrunde liegende Reaktionsablauf ist in nachfolgendem Schema dargestellt:

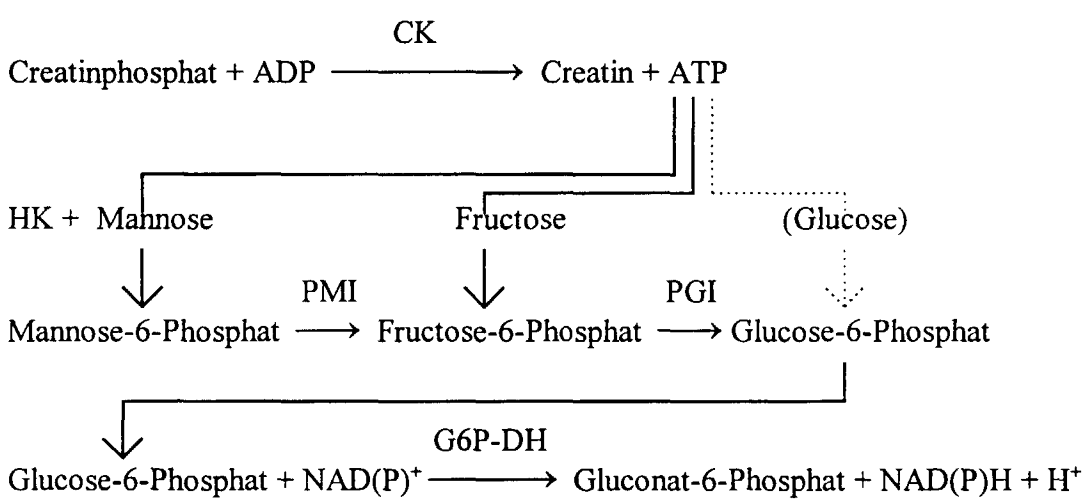


Im Gegensatz zu Glucose wird überraschenderweise Fructose oder Mannose von Glucose-6-phosphat-Dehydrogenase in Gegenwart von nicht reduziertem Coenzym, wie $NAD^+$ oder $NADP^+$, nicht oder nur in äußerst geringfügigem Umfang umgesetzt, so daß bei Verwendung dieser Hexosen eine vorzeitige Bildung von NADH oder NADPH im CK-Reagenz vermieden wird.

Die erforderlichen Reaktionskomponenten sowie gegebenenfalls weitere Hilfsstoffe können in einem einzigen Reagenz vereint oder verteilt auf mehrere Teilreagenzien vorliegen, wobei im Falle der Verteilung auf mehrere Teilreagenzien Creatinphosphat im für den Start der Creatinkinase-Nachweisreaktion verwendeten Teilreagenz enthalten ist.

Erfindungsgemäß beträgt die Konzentration an Fructose bzw. Mannose im Reagenz etwa 0,05 bis 200 mmol/l, bevorzugt 0,1 bis 50 mmol/l. Phosphoglucose-Isomerase und Phosphomannose-Isomerase werden zu Volumenaktivitäten von mindestens 0,1 U/ml, bevorzugt zwischen etwa 0,5 bis 50 U/ml, insbesondere zwischen etwa 1 bis 30 U/ml, eingesetzt. Alle übrigen Komponenten sowie gegebenenfalls weitere Hifsstoffe, einschließlich der gegebenenfalls zuzusetzenden 6-Phosphogluconolactonase und Gluconat-6-phosphat-Dehydrogenase, werden in für den Fachmann üblichen Konzentrationen verwendet. Bei den angegebenen Konzentrationen handelt es sich jeweils um Angaben für die fertige bzw. aus verschiedenen Teilreagenzien vereinigte Bestimmungslösung (Endkonzentrationen).

Das erfindungsgemäße wäßrige Reagenz zeichnet sich durch besondere Stabilität nach Lagerung bei über 0°C aus. So wurden beispielsweise durch teilweisen oder vollständigen Ersatz von Glucose durch Fructose und/oder Mannose und Lagerung entsprechender Reagenzien bei 4 bis 8 °C über einen Zeitraum von bis zu 12 Monaten CK-Wiederfindungen von annähernd 100%, verglichen mit denjenigen mit einem frisch hergestellten Reagenz erhaltenen, festgestellt. Im Gegensatz dazu weisen bekannte CK-Flüssigreagenzien Stabilitäten sehr viel schlechterer Qualität auf. Dies gilt auch für CK-Reagenzien gemäß EP 0 774 514, denen anstelle von N-Acetylcystein eine andere Sulfhydrylverbindung und ein Phosphin zugesetzt werden. Derartige Zusätze sind darüber hinaus nicht geeignet, das der vorliegenden Erfindung zugrundeliegende Problem zu lösen, d.h. die vorzeitige Bildung von NADH oder NADPH zu verhindern.

Erläuterung der Abbildungen:

Abbildung 1: Zugabe von $NADP^+$ (Reagenz B) zu einer Glucose-6-phosphat-Dehydrogenase sowie Glucose (Stand der Technik) bzw. Fructose oder Mannose enthaltenden Lösung (erfindungsgemäß).

Abbildung 2: Creatin-Kinase-Methodenvergleich (Boehringer Mannheim/Hitachi 717; 37°C); Stand der Technik entsprechend Beispiel 2, erfindungsgemäß entsprechend Beispiel 3. (x: Humanseren (10); Δ: Kontrollseren (6)).

Abbildung 3: Creatin-Kinase-Methodenvergleich (Boehringer Mannheim/Hitachi 717; 37°C; Stand der Technik entsprechend Beispiel 2, erfindungsgemäß entsprechend Beispiel 4. (x: Humanseren (10); Δ: Kontrollseren (6)).

Die nachstehenden Beispiele erläutern die Erfindung weiter.

<u>Beispiel 1:</u> Umsetzung verschiedener Hexosen mit Glucose-6-phosphat-Dehydrogenase in Gegenwart von $NADP^+$

Reagenzien:

| | | |
|---|---|---|
| Reagenz A: | Imidazol • HCl (pH 6,6) | 110 mmol/l |
| | Hexose* | 50 mmol/l |
| | G6P-DH | 10 U/ml |
| Reagenz B: | $NADP^+$ (in dest. $H_2O$) | 25 mmol/l |

*)Glucose, Fructose oder Mannose

Testansatz: Es wurden in einer Küvette mit einer Schichtdicke von 1 cm jeweils 1,0 ml Reagenz A mit 0,1 ml Reagenz B vermischt und mit einem Spektralphotometer bei 37 °C und 340 nm der Extinktionsverlauf über ca. 40 min verfolgt.

Wie aus Abbildung 1 zu ersehen ist, erfolgt mit Glucose eine signifikante NADPH-Bildung, während mit Fructose oder Mannose keine Extinktionsveränderung meßbar ist.

Beispiel 2: Bestimmung der Creatin-Kinase in Serum mit herkömmlichem Reagenz ("CK NAC aktiviert", Boehringer Mannheim)

2.1 Reagenzzusammensetzung:

| | | |
|---|---|---|
| Teilreagenz 1 ("R1"): | Imidazol-Acetat (pH 6,7) | 110 mmol/l |
| | Glucose | 20,5 mmol/l |
| | Mg-Acetat | 10 mmol/l |
| | EDTA | 2 mmol/l |
| | Adenosin-5'-diphosphat | 2,5 mmol/l |
| | Adenosin-5'-monophosphat | 6,1 mmol/l |
| | Di-adenosin-pentaphosphat | 12 µmol/l |
| | $NADP^+$ | 2,5 mmol/l |
| | N-Acetylcystein | 25 mmol/l |
| | Hexokinase | 5 U/ml |
| | G6P-DH | 3 U/ml |
| Teilreagenz 2 ("R2"): | Imidazol-Acetat (pH 7,5) | 25 mmol/l |
| | Glucose | 20,5 mmol/l |
| | EDTA | 2 mmol/l |
| | Mg-Acetat | 10 mmol/l |
| | Creatinphosphat | 184 mmol/l |

2.2 Durchführung der Bestimmung:

1. Die Messungen erfolgten mit einem Boehringer Mannheim/Hitachi 717-Analysenautomaten (Temperatur 37 °C; Wellenlängen 405/340 nm; 250 µl Teilreagenz 1, 50 µl Teilreagenz 2, 7 µl Probe; Meßmethode Kinetik A 5-35-50). Zur Kalibration wurde der "Calibrator for Automated Systems", Boehringer Mannheim GmbH, Best.-Nr. 759 350, eingesetzt.

Beispiel 3: Bestimmung der Creatin-Kinase in Serum mit dem erfindungsgemäßen Reagenz; vollständiger Ersatz von Glucose durch Fructose:

3.1 Reagenzzusammensetzung:

| | | |
|---|---|---|
| Teilreagenz 1 ("R1"): | Imidazol-Acetat (pH 6,7) | 110 mmol/l |
| | Fructose | 25 mmol/l |
| | Mg-Acetat | 10 mmol/l |
| | EDTA | 2 mmol/l |
| | Adenosin-5'-Diphosphat | 2,5 mmol/l |
| | Adenosin-5'-monophosphat | 6,1 mmol/l |
| | Di-Adenosin-Pentaphosphat | 12 µmol/l |
| | $NADP^+$ | 2,5 mmol/l |
| | N-Acetylcystein | 25 mmol/l |
| | Hexokinase | 5 U/ml |
| | Phosphoglucose-Isomerase | 10 U/ml |
| | G6P-DH | 3 U/ml |
| Teilreagenz 2 ("R2"): | Imidazol-Acetat (pH 7,5) | 25 mmol/l |
| | Fructose | 25 mmol/l |
| | EDTA | 2 mmol/l |
| | Mg-Acetat | 10 mmol/l |
| | Creatinphosphat | 184 mmol/l |

3.2 Durchführung der Bestimmung: Wie unter Beispiel 2.

Wie aus Abbildung 2 zu ersehen ist, liefert die Messung der CK in Human- bzw. Kontrollseren nach Beispiel 3 die gleichen Ergebnisse wie die nach Beispiel 2 erhaltenen.

Beispiel 4: Bestimmung der Creatin-Kinase in Serum mit dem erfindungsgemäßen Reagenz; Ersatz von Glucose durch Fructose im Teilreagenz 1:

4.1 Reagenzzusammensetzung:

| | | |
|---|---|---|
| Teilreagenz 1 ("R1"): | Imidazol • HCl (pH 6,7) | 110 mmol/l |
| | Fructose | 25 mmol/l |
| | Mg-Acetat | 10 mmol/l |
| | EDTA | 2 mmol/l |
| | Adenosin-5'-Diphosphat | 2,5 mmol/l |
| | Adenosin-5'-monophosphat | 6,1 mmol/l |
| | Di-Adenosin-Pentaphosphat | 10 μmol/l |
| | $NADP^+$ | 2,5 mmol/l |
| | N-Acetylcystein | 25 mmol/l |
| | Hexokinase | 5 U/ml |
| | Phosphoglucose-Isomerase | 10 U/ml |
| | G6P-DH | 3 U/ml |
| Teilreagenz 2 ("R2"): | Imidazol • HCl (pH 7,5) | 25 mmol/l |
| | Glucose | 20,5 mmol/l |
| | EDTA | 2 mmol/l |
| | Mg-Acetat | 10 mmol/l |
| | Creatinphosphat | 184 mmol/l |

4.2 Durchführung der Bestimmung: Wie unter Beispiel 2.

Wie aus Abbildung 3 zu ersehen ist, liefert die Messung der CK in Human- bzw. Kontrollseren nach Beispiel 4 die gleichen Ergebnisse wie die nach Beispiel 2 erhaltenen.

Resultate entsprechender Qualität werden ebenfalls erhalten; wenn anstelle von Fructose Mannose im Teilreagenz 2 und/oder Teilreagenz 1 nach Beispiel 3 oder 4 verwendet und neben der Phosphoglucose-Isomerase zusätzlich noch Phosphomannose-Isomerase (10 U/ml) zu Teilreagenz 1 zugesetzt wird.

Gleichfalls ist es möglich, Glucose im Reagenz nach Beispiel 1 vollständig durch beispielsweise Galactose, die Hexokinase durch Galactokinase und Glucose-6-Phosphat-Dehydrogenase durch Galactose-6-Phosphat-Dehydrogenase zu ersetzen, ohne daß die Wiederfindungsrate der CK entscheidend beeinflußt wird.

## Patentansprüche

1. Verfahren zur enzymatischen Bestimmung von Creatin-Kinase in biologischem Probenmaterial durch Bildung von Adenosin-5'-triphosphat aus Creatinphosphat und Adenosin-5'-diphosphat und Nachweis des gebildeten Adenosin-5'-triphosphat unter Verwendung von Glucose, einer Hexokinase, Hexose-6-phosphat-Dehydrogenase und $NAD^+$ oder $NADP^+$, dadurch gekennzeichnet, daß als Substrat für die Hexokinase zusätzlich oder anstelle von Glucose eine von Glucose unterschiedliche, enzymatisch phosphorylierbare Hexose verwendet wird und das in der Hexokinase-Reaktion gebildete, von Glucose-6-phosphat unterschiedliche Hexose-6-phosphat entweder direkt mittels einer von Glucose-6-phosphat-Dehydrogenase unterschiedlichen Hexose-6-Phosphat-Dehydrogenase, oder nach enzymatischer Isomerisierung, als Glucose-6-phosphat mittels der Glucose-6-phosphat-Dehydrogenase bestimmt wird.

2. Verfahren nach Anspruch 1, dadurch gekennzeichnet, daß die erforderlichen Reaktionskomponenten und gegebenenfalls weitere Hilfsstoffe in einem einzigen Reagenz vereint oder verteilt auf mehrere Teilreagenzien vorliegen, wobei im Falle der Verteilung auf mehrere Teilreagenzien Creatinphosphat im für den Start der Creatinkinase-Nachweisreaktion verwendeten Teilreagenz enthalten ist.

3. Verfahren nach Anspruch 2, dadurch gekennzeichnet, daß zumindest in dem für die Erfassung der gegebenenfalls in der Probe enthaltenen Adenylat-Kinase-Aktivität und/oder für die Aktivierung der Creatin-Kinase verwendeten Teilreagenz die Glucose vollständig durch eine von Glucose unterschiedliche Hexose ersetzt ist und eine oder mehrere Hexose-6-phosphat-Isomerasen enthalten sind.

4. Verfahren nach einem der Ansprüche 1 bis 3, dadurch gekennzeichnet, daß als von Glucose unterschiedliche Hexose Fructose und/oder Mannose und als Hexose-6-phosphat-Isomerase Phosphoglucose-Isomerase oder ein Gemisch bestehend aus Phosphomannose-Isomerase und Phosphoglucose-Isomerase verwendet wird.

5. Verfahren nach einem der Ansprüche 1 bis 4, dadurch gekennzeichnet, daß die Bestimmung in Gegenwart von 6-Phosphogluconolactonase und Gluconat-6-phosphat-Dehydrogenase erfolgt.

6. Reagenz zur Bestimmung von Creatin-Kinase in biologischem Probenmaterial, enthaltend in wäßrigem, gepufferten Medium Magnesium- oder Mangan-Ionen, Creatinphosphat, Adenosin-5'-diphosphat, eine Hexokinase, eine Creatin-Kinase-aktivierende Verbindung, einen oder mehrere Adenylat-Kinase-Inhibitoren sowie $NAD^+$ oder $NADP^+$, dadurch gekennzeichnet, daß es zusätzlich zu oder anstelle von Glucose eine von Glucose unterschiedliche, enzymatisch phosphorylierbare Hexose sowie entweder eine von Glucose-6-phosphat-Dehydrogenase unterschiedliche Hexose-6-phosphat-Dehydrogenase oder Glucose-6-phosphat-Dehydrogenase in Kombination mit einer oder mehreren Hexose-6-phosphat-Isomerasen enthält.

7. Reagenz nach Anspruch 6, dadurch gekennzeichnet, zusätzlich zu oder anstelle von Glucose Fructose und/oder Mannose sowie Phosphoglucose-Isomerase oder ein Gemisch bestehend aus Phosphomannose-Isomerase und Phosphoglucose-Isomerase enthalten sind.

8. Reagenz nach Anspruch 6 oder 7, dadurch gekennzeichnet, daß es aus mindestens zwei Teilreagenzien besteht und daß die von Glucose unterschiedliche Hexose sowie Hexose-6-phosphat-Isomerase bzw. -Isomerasen in dem für die Aktivierung der Creatinkinase und/oder dem für die Bestimmung der im Probenmaterial neben der Creatin-Kinase gegebenenfalls vorhandenen Adenylat-Kinase vorgesehenen Teilreagenz enthalten sind.

9. Reagenz nach einem der Ansprüche 6 bis 8, dadurch gekennzeichnet, daß Fructose und/oder Mannose und als Hexose-6-phosphat-Isomerasen Phosphoglucose-Isomerase oder ein Gemisch aus Phosphomannose-Isomerase und Phosphoglucose-Isomerase enthalten sind.

10. Reagenz nach einem der Ansprüche 6 bis 9, dadurch gekennzeichnet, daß das Reagenz bzw. ein Teilreagenz 6-Phosphogluconolactonase und Gluconat-6-phosphat-Dehydrogenase enthält.

11. Reagenz nach einem der Ansprüche 6 bis 10, dadurch gekennzeichnet, daß die Konzentration der von Glucose unterschiedlichen Hexose bzw. Hexosen in der Testlösung etwa 0,05 bis 200 mmol/l beträgt.

12. Reagenz nach einem der Ansprüche 6 bis 11, dadurch gekennzeichnet, daß die Aktivität der Hexose-6-phosphat-Isomerasen jeweils mindestens etwa 0,1 U/ml beträgt.

Abbildung 1

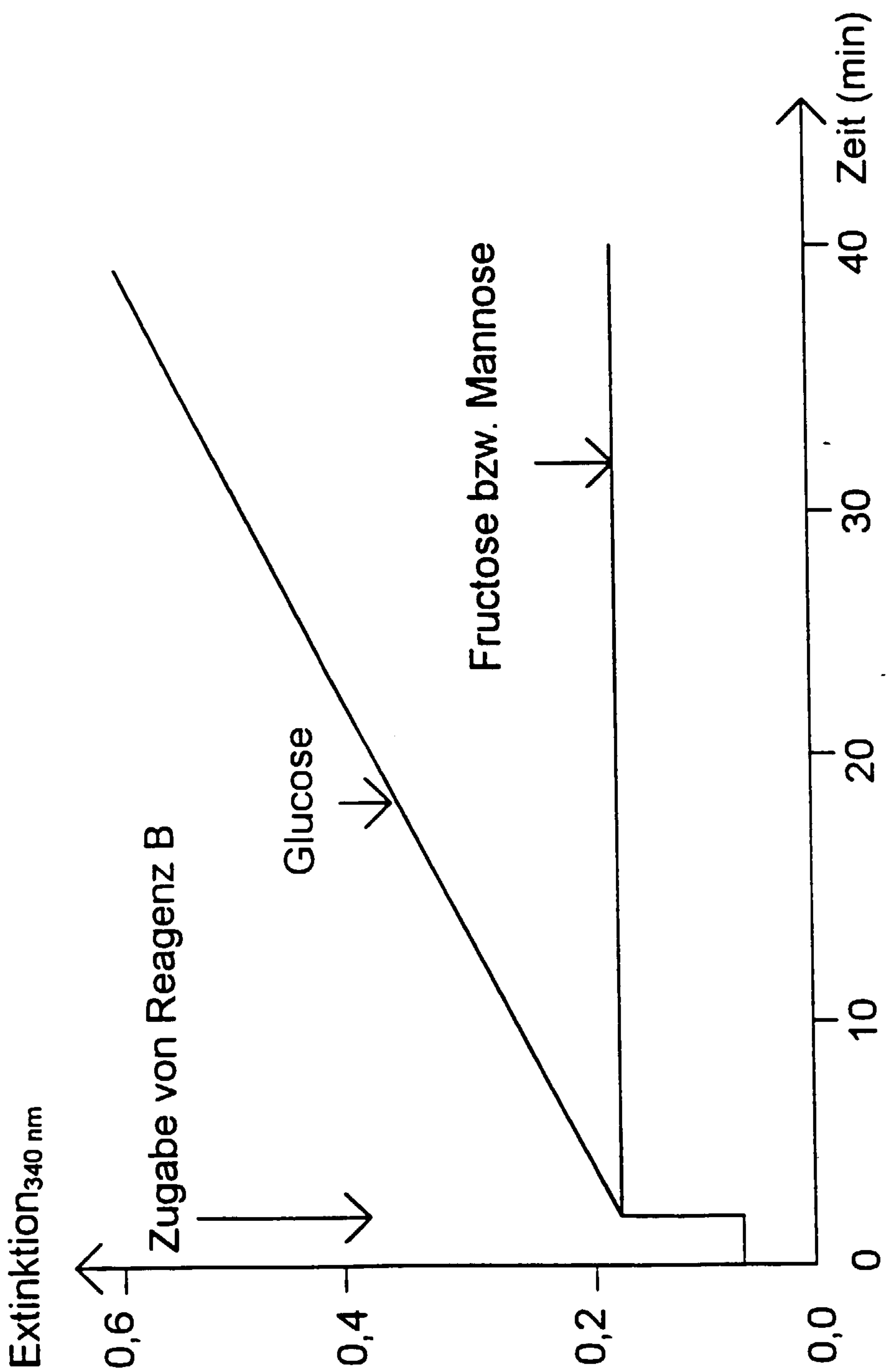
Extinktion340 nm
0,6
0,4
0,2
0,0
Zugabe von Reagenz B
Glucose
Fructose bzw. Mannose
0
10
20
30
40
Zeit (min)

Abbildung 2

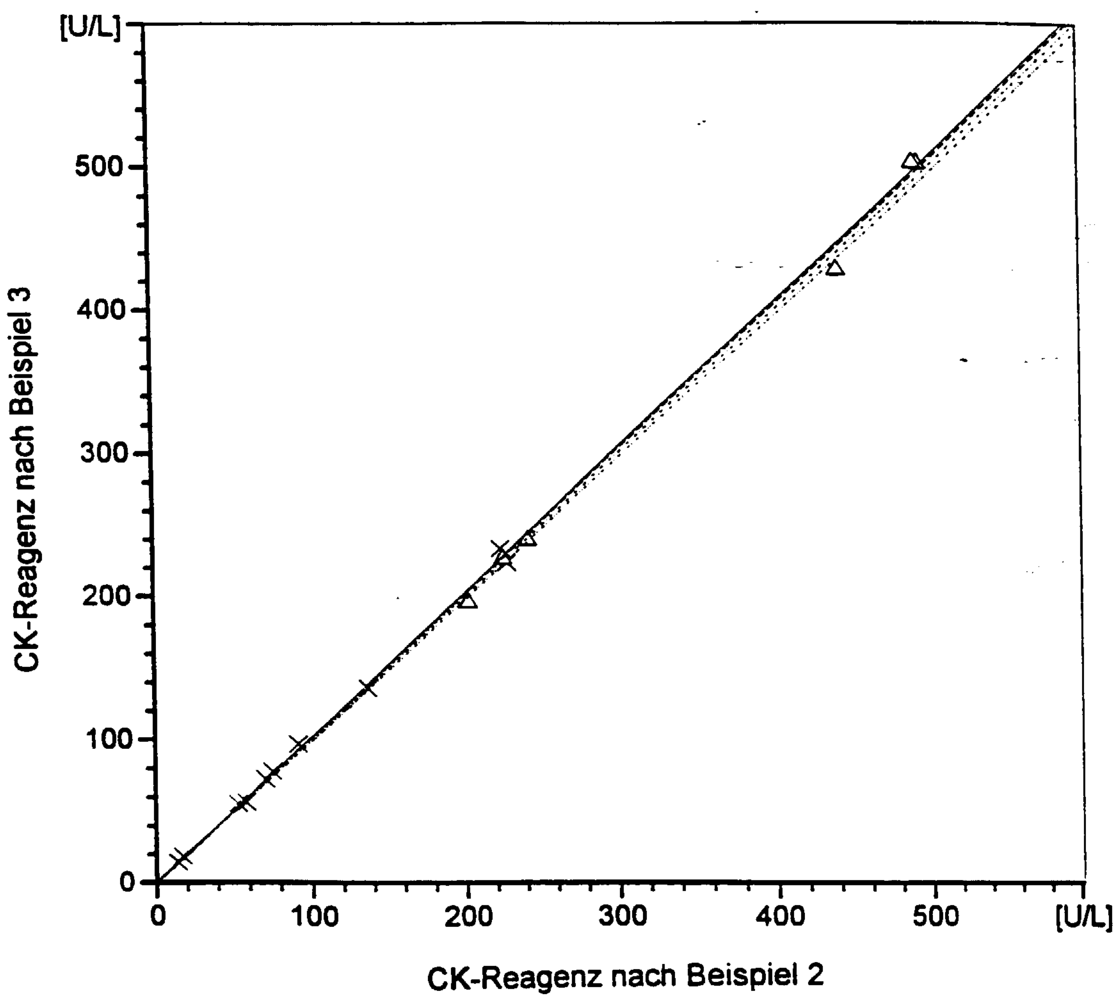
[U/L]
500
400
300
200
100
0
CK-Reagenz nach Beispiel 3
0
100
200
300
400
500
[U/L]
CK-Reagenz nach Beispiel 2

Abbildung 3

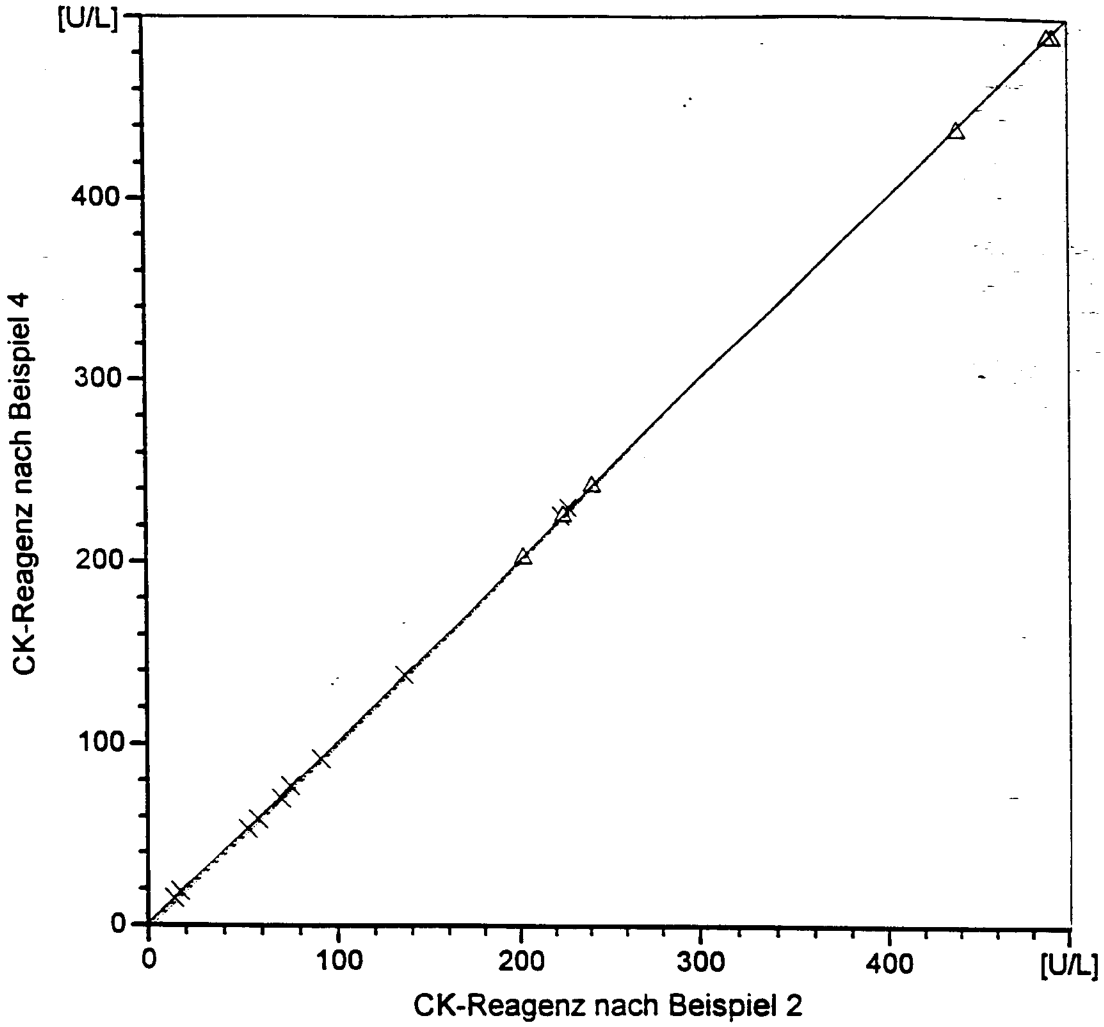
[U/L]
400
300
200
100
0
CK-Reagenz nach Beispiel 4
0
100
200
300
400
[U/L]
CK-Reagenz nach Beispiel 2